# EUROPEAN PATENT SPECIFICATION

(11) **EP 0 292 321 B1**
(45) Date of publication and mention of the grant of the patent: **31.08.1994**
(21) Application number: 88304608.8
(22) Date of filing: 20.05.1988
(51) Int. Cl.: C12N 9/96, A61K 37/50, C12N 9/02

(54) **Acylated derivative of superoxide dismutase and composition containing same**
Azyliertes Derivat von Superoxid-Dismutase und dieses enthaltende Zusammensetzung
Dérivé acétylé de la superoxyde dismutase et composition la contenant

(30) Priority: 21.05.1987 JP 122567/87; 09.09.1987 JP 223971/87
(43) Date of publication of application: 23.11.1988
(73) Proprietor: SUNTORY LIMITED, Kita-ku, Osaka-shi, Osaka-fu 530 (JP); Inoue, Masayasu, Kumamoto-shi Kumamoto (JP)
(72) Inventor: Inoue, Masayasu, Kumamoto-shi Kumamoto (JP); Ando, Yukio, Kumamoto-shi Kumamoto (JP); Utsumi, Kozo, Kouchi-shi Kouchi (JP); Utsumi, Toshihiko, Yamaguchi-shi Yamaguchi (JP)
(74) Representative: Ford, Michael Frederick

(56) References cited:
- EP-A- 0 200 467
- EP-A- 0 210 761
- DE-A- 2 539 778
- CHEMICAL ABSTRACTS, vol. 106, no. 5, February 2, 1987, Columbus, Ohio, USA F.MARMOCCHI et al. "Rever- sible acylation of copper, zinc superoxide dismutase
- by citraconic anhydride" page 203, left column, Abstract-no. 29 194j

## Description

The present invention relates to novel derivatives of superoxide dismutase (referred to hereinafter as "SOD"), more particularly acylated superoxide dismutases (referred to hereinafter as "Ac-SOD").

Superoxide dismutase (SOD) is an enzyme which cleaves and eliminates a superoxide (active oxygen atom) harmful to a living body, and exists in all viscera and intracellular fractions (cytoplasm and mitochondrion). But, this protective mechanism is extremely weak at the cell membrane and outside tissue cells, so very dangerous oxidative diseases can sometimes occur at those portions. Previously, to remedy such a disease due to oxidatively injured tissue, SOD was administrated intravenously or topically. But SOD administrated as above does not exhibit a sufficient protective action, because of instability in the body and a short half-life (about 4-6 minutes).

Recently, SOD has been chemically modified in various ways, to extend the half-life thereof in blood. For example, SOD has been modified with polyoxyethylene glycol (JP-A-61-249388), polymeric dextran [W. F. Petrone et al. Proc. Natl. Acad. Sci. USA 77, 1159 (1980)] and insulin (JP-A-58-32826).

Nevertheless, the modified SOD's are still defective, in that the object of developing the SOD's modified as above was to enable them to be administrated intravenously or intramuscularly, whereby the protection of oxidative injuries of the tissue is carried out in the blood. The oxidative injuries (for example, inflammation) of the tissue, include not only diseases which need systemic treatment, but also those occurring in the eyes (keratitis and keratohelcosis), skin (burns, various dermatitides, etc), and mucous membranes of the oral cavities and digestive tracts, such as the stomach (various inflammations and ulcers). Further, a biotoxin of an active oxygen molecular species (such as superoxide) mainly occurs on the membrane, and thus it is important to cleave and eliminate harmful oxygen species, particularly around cell membranes, of the latter diseases.

Although the conventionally modified SOD's are stable in blood, they are giant molecules and therefore have disadvantages e.g. in that wetting diffusion into a gap between tissues is reduced, and action after approaching or binding to the surface of the cell membrane is impossible.

US-A-4017605 (equivalent to DE-A-2539778) describes anti-inflammatory orgotein derivatives having superoxide dismutase activity, in which amino groups are acylated. Use of at least ten acyl groups, each having up to 8 carbon atoms, is recommended per molecule.

The development of a novel SOD derivative, which acts after approaching or binding to the cell membrane where the active oxygen molecule species exerts toxicity, is desired. Particularly, for the inhibition or decrease of oxidative injuries in tissues such as the eye, skin, and mucous membranes of the oral cavity and digestive tracts, and thus there is a strong demand for a development of a SOD derivative which is enzymologically stable, does not exert side-effects, and is binding to the cell membrane; i.e., which may be bound to the surface of the cell membrane, or rapidly metabolized and excreted when insorpted in blood, and exert very little side-effects.

Further, in the administration of SOD or a derivative thereof to diseases of the cornea, it is desirable to use an instillation, which is a routine and non-attacking method in ophthalmology. But, SOD and the conventional derivatives thereof are rapidly removed from the surface of cornea by lacrima, and thus cannot exert any action thereon. Therefore, there is a desire for the development of a preparation which will remain on the corneal surface for a long term and effectively act thereon.

After conducting various research projects to remedy the above-mentioned problems, the present inventors found that derivatives obtained by acylating SOD with various fatty acids can bind to the surfaces of various tissue cells and a double layer of lipids to eliminate superoxide present there, or be rapidly metabolized in blood and excreted therefrom, and it is not a giant molecule. Further, the present inventors have found that the above-mentioned new derivative of SOD has pharmaceutically useful properties which non-modified SOD and conventional SOD derivatives do not exhibit. Namely, the new SOD derivatives can be bound and fixed to the surfaces of cell membranes of the cornea, skin, mucous membranes of the digestive tract, erythrocyte and the like, and remarkably inhibit or reduce various inflammatory diseases occurring at those portions for a long term. According to the present inventors, the above-mentioned new derivatives of SOD have a particularly high affinity to a lipid layer in an epithelial cell membrane of the cornea.

In one aspect, the present invention provides an acylated derivative of superoxide dismutase having the general formula 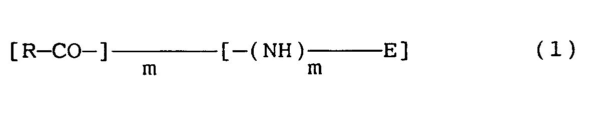
where R-CO- represents an acyl group of a saturated or unsaturated fatty acid having 10 to 16 carbon atoms, m is an integer of 4 to 8, and (NHₘ)E represents a group of superoxide dismutase (NH₂)ₘE.

In other aspects, the invention provides compositions containing such derivatives, and particularly pharmaceutical compositions for treating inflammation such as keratohelcosis.

In other aspects, the invention provides compositions containing such a derivative, and particularly a pharmaceutical composition for treating inflammation such as keratohelcosis.

Specifically, one aspect provides a composition containing an effective amount of the acylated derivative of superoxide dismutase, as a preventive or curing agent for a disease caused by a direct or indirect harmful action of superoxide, as a stabilizing agent for a blood product, or as a modifying agent for a surface of a medical vessel, together with a suitable carrier.

Further, the present invention provides a pharmaceutical composition for treating keratohelcosis, comprising the acylated derivative of superoxide dismutase together with a pharmaceutical acceptable carrier.

### BRIEF EXPLANATION OF THE DRAWINGS

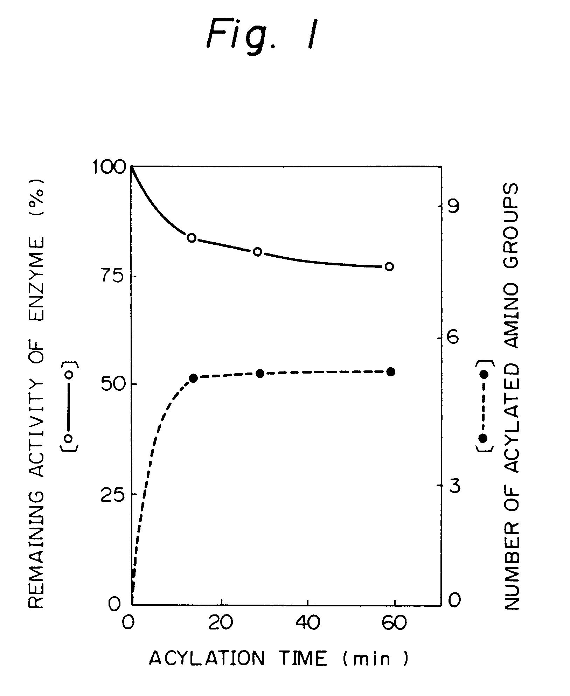

Figure 1 is a graph showing the relationship between an acylation time with a number of acylated amino groups and the remaining activity of enzyme in an acylation of human SOD;

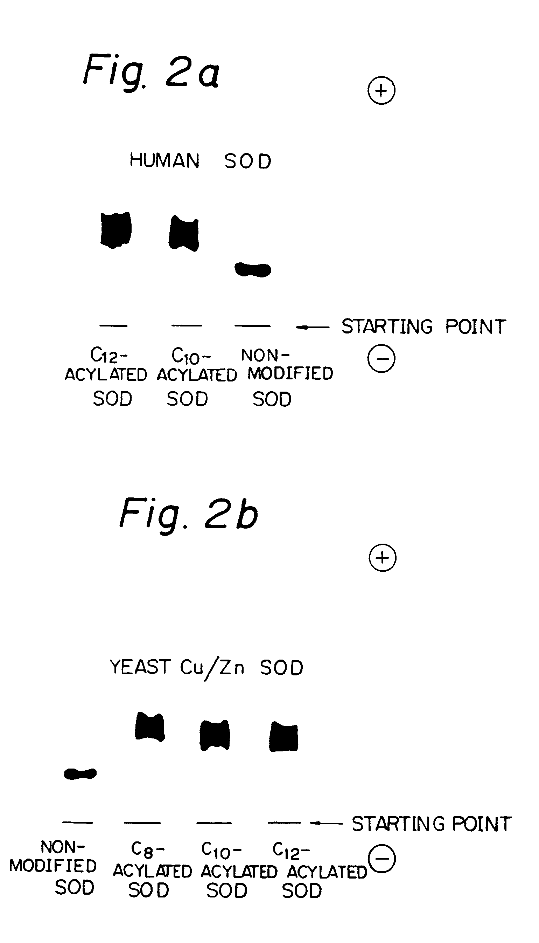

Figures 2a and 2b illustrate the results of an electrophoresis of agarose gel for non-modified SOD's and acylated SOD's from human and yeast, respectively;
Figure 3 illustrates amounts of non-modified SOD's and acylated SOD's bound to erythrocytes in serum and PBS;
Figures 4a and 4b are chromatograms of Sephadex G-200 column of reaction products of non-modified SOD and acylated SOD (yeast SOD modified with myristic acid) with liposome, respectively;
Figure 5 illustrates activities of enzymes (SOD's) for non-modified SOD and acylated SOD bound to liposome; and
Figure 6 illustrates metabolisms for non-modified SOD and acylated SOD (modified with caprylic acid) in blood.

### DESCRIPTION OF THE PREFERRED EMBODIMENTS

The acylated derivative of superoxide dismutase (Ac-SOD) according to the present invention typically has the structure of the general formula 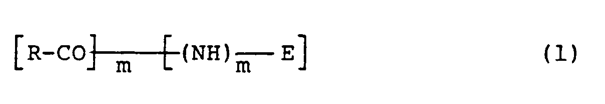
wherein R-CO- represents an acyl group of a saturated or unsaturated, preferably saturated or mono-unsaturated fatty acid having 10 to 16, more preferably 12 carbon atoms, m is an integer of 4 to 8, and 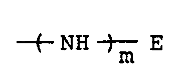
represents a group of superoxide dismutase

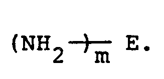

Any SOD's from organisms such as animals (e.g., human, cattle), plants and microorganisms may be employed to prepare the Ac-SOD of the present invention. SOD's used as a starting material may be naturally occurring products or those obtained by gene manipulation.

The method for modifying SOD with acyl groups is not limited. For example, when a myristic acid is used as an acylating agent, myristyl chloride may be reacted with N-hydroxysuccinimide to form an active myristic acid ester. Then, the active ester is added dropwise to and reacted with SOD in 0.1M NaHCO₃ solution to prepare myristylated SOD.

In the above general formula (1), the acyl group may be derived from any saturated and unsaturated fatty acids having an odd number of carbon atoms. Preferably however, the acyl group is derived from saturated fatty acid having an even number of carbon atoms, such as capric acid (C₁₀), lauric acid (C₁₂), myristic acid (C₁₄), palmitic acid (C₁₆) or the like. This is because saturated fatty acids having an even number of carbon atoms are naturally present in a body and can be cleaved in a normal lipometabolism to generate energy, which is preferable from the viewpoint of toxicity.

The number (m) of acyl groups may be controlled by various reaction conditions between the active ester of fatty acid and SOD. In this connection, it should be understood that the superoxide dismutase proper (E) may contain amino groups other than those represented by (̵NH₂)ₘ.

It is believed that the acyl group of Ac-SOD has an affinity to a hydrophobic region in the surface layer of the cell, and the acyl chain reversibly binds to the surface of the cell membrane, to thereby exert the described useful effects.

In comparison with the natural (non-modified) SOD, Ac-SOD according to the present invention has a higher affinity to the lipid in the cell membrane, whereby SOD per se can be topically concentrated at the locale of the disease, in a stable state. Further, Ac-SOD of the present invention may be stably maintained on eye, skin, and mucous membrane of oral cavity and digestive tract including stomach. Therefore, Ac-SOD according to the present invention can be used, for example, for a protection of the diseases caused by harmful actions of active oxygen (superoxide), particularly, pulmonary or ophthalmic diseases or radiation hazards (particularly, cutaneous ulcers); an impediment to the progress of diseases caused by a peroxidation of lipid, for example, senescence; protection of the cell membrane; or as a preventive or curing agent for wide range of inflammatory diseases, such as dermatitis, burns, inflammation of the oral cavity or digestive tracts (e.g., esophagitis, gastritis, ulcers in the digestive tract).

Further, the Ac-SOD of the present invention can provide medicine having a high safety factor when used in combination with other medicines (for example, an anticancer agent having strong side effects) which exhibit side effects caused by the generation of superoxide, or skin disease caused by superoxide generated by sunlight or radiation. The present Ac-SOD may be used as a stabilizing and preserving agent for a blood product, particularly a concentrated erythrocyte product.

When the Ac-SOD according to the present invention is taken up in the blood although intended to exert action on the surface of the cell membrane, it is rapidly metabolized and excreted from the kidney to the urine, similar to a non-modified SOD, and therefore, it is believed that there is little chance of any side effect being caused by a retention of the As-SOD for a long term in the body. When fatty acids the same as those constituting the human body are used for the acylation of SOD in the present invention, the Ac-SOD of the present invention will not produce metabolites providing side effects and toxicity.

The Ac-SOD according to the present invention can be given for the above-mentioned diseases in various forms of medical preparations and by various administration methods. For example, a preparation produced by dissolving Ac-SOD in a 5% glucose solution or physiological saline may be administrated by instillation or venoclysis. A mixture of an appropriate amount (e.g., 0.2 to 10 mg/ml) of Ac-SOD according to the present invention and a 10 - 30% glycerol solution may be applied on the skin or to a local disease spot.

A preparation produced by mixing a lyophilized powder of Ac-SOD and an appropriate amount of lactose may be used as an internal medicine. When incorporated into a blood product such as a concentrated erythrocyte product, 1 to 20 mg of Ac-SOD may be added to 200 - 400 ml of that product. Further, the Ac-SOD solution may be brought into contact with the surface of the medical vessel, thereby adsorbing Ac-SOD to the surface, and used as the modifying agent therefor.

A pharmaceutical composition for treating keratohelcosis, containing Ac-SOD as an effective ingredient, may be prepared as follows. Ac-SOD is modified with acylation, but it retains protein properties. Therefore, care should be taken when stabilizing Ac-SOD against microorganisms in the aqueous instillation solution. To ensure storage stability, preferably a preservative known in ophthalmology, for example, benzalkonium chloride, cetylpyridinium chloride, chlorobutanol or thyronesal, individually or in combination thereof is added in an amount of 0.001 - 0.1% with respect to the total preparation. The osmotic pressure ratio is preferably about 1. As an isotonic agent, sodium chloride, mannitol, boric acid or the like may be used. The pH of the composition is preferably 7 - 8 and as a buffer, phosphate, borate, a salt of an organic acid or the like can be used as in the conventional instillation solution. Further, as occasion demands, a safe thickener, and a protein stabilizing agent such as dextrin or cyclodextrin may be incorporated.

A desired instillation composition (0.1% to 3%) may be prepared by dissolving lyophilized Ac-SOD in distilled water for injection which contains the auxiliary additives; filtrating, and then sterilizing. The additives may be added to the distilled water in any order. When the lyophilized Ac-SOD is solubilized in water immediately prior to use thereof, it may be directly dissolved in the physiological saline for injection without the storage stabilizer.

### EXAMPLES

The present invention now will be further illustrated by, but is by no means limited to, the following Examples.

### Example 1: Acylation of Yeast SOD

N-hydroxysuccinimide (36 mmole) was dissolved in 20 ml of pyridine. To this solution, myristyl chloride (36 mmole) dissolved in 50 ml of pyridine was added and stirred at a room temperature for 12 hours. After adding 100 ml of ethyl acetate, the reaction solution was extracted, and washed with 1N HCl, a saturated sodium hydrogencarbonate solution, and a saturated sodium chloride solution. The residual water was removed with sodium sulfate, and the precipitate resulting from the above procedures was filtered off. The resulting filtrate was concentrated to dryness and crystallized from hexane and then ethanol, to obtain an active ester of myristic acid.

Two mg (0.061 µmole) of yeast SOD were dissolved in 1 ml of 0.1 M sodium hydrogencarbonate solution (pH 8.0). To this solution, 0.2 mg (0.61 µmole) of the active ester of myristic acid dissolved in 0.1 ml of dimethyl sulfoxide (DMSO) was added, and a reaction was carried out under stirring at 25°C for 24 hours.

After the reaction was completed, the reaction product was treated by Sephadex G-25 column chromatography (ø1 x 30 cm) equilibrated with a 20 mM phosphate buffer containing 100 mM NaCl (pH 7.4) [referred to as "PBS"], and a dialysis of active fractions to the PBS was performed to obtain an SOD modified with myristic acid.

### Example 2: Binding Property of Ac-SOD to Erythrocyte

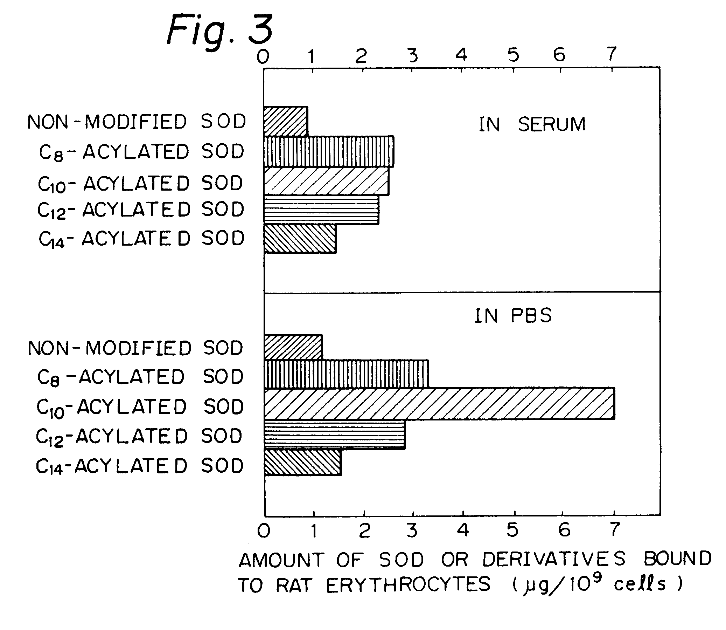

Rat erythrocyte and 20 µg of human SOD modified with radioactively labeled capric, lauric or myristic acid were incubated in 1 ml of PBS or serum at 37°C for 30 minutes, washed twice with 2 ml of PBS, and then the amount of the enzymes bound to the erythrocyte was measured. The results are shown in Figure 3.

The amount of the acylated SOD bound to the rat erythrocyte was as much as two to seven times that of non-modified SOD. The binding property was higher in the serum free condition (i.e., in PBS; see the lower half of Figure 3).

### Example 3: Binding Property of Ac-SOD to Liposome

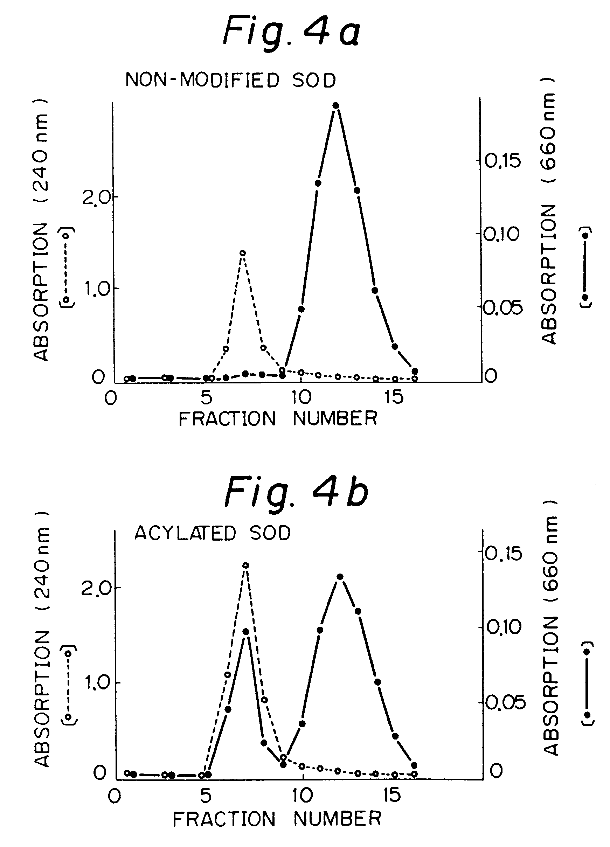

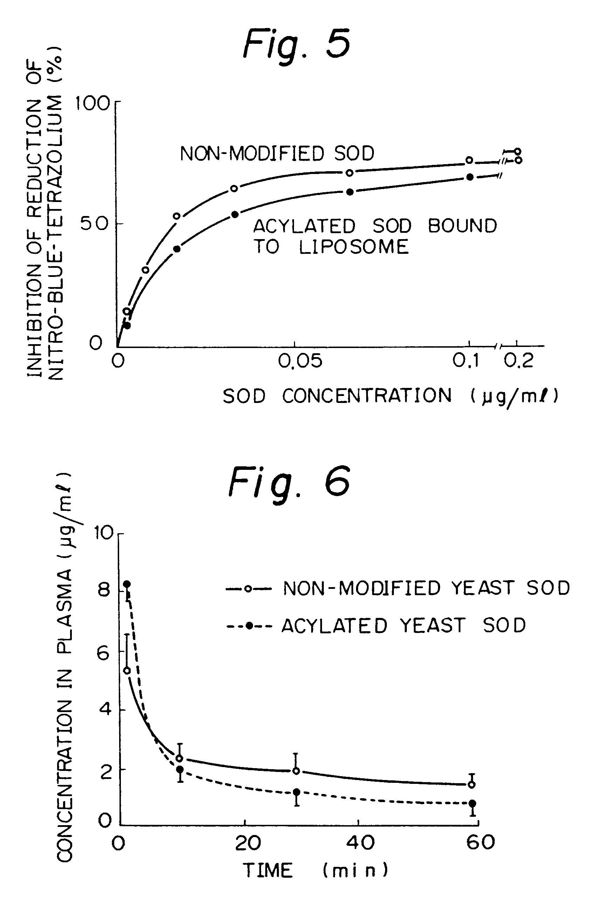

In 0.5 ml of PBS, 2.5 mg of dipalmitoryl-L-α-phosphatidyl choline (DPPC)-liposome were suspended to form a liposome. Yeast SOD or yeast SOD modified with myristic acid (0.5 mg) dissolved in 0.5 ml of PBS was then added, and reaction was carried out at 25°C for 30 minutes. Purification was then performed by gel filtration with Sephadex G-200 (ø1 x 30 cm). Figure 4 shows the column chromatogram of Sephadex G-200. In Figure 4, the absorption at 240 nm is that by liposome, and the absorption at 660 nm is that by protein (the method of Lowry et al). Figure 5 illustrates a comparison of activities between non-modified SOD and SOD modified with myristic acid and then bound to liposome. Although the acylated SOD was bound to liposome, the enzymatic activity thereof was comparable to that of non-modified SOD. These results mean that when Ac-SOD according to the present invention is bound to the surface of the cell membrane, the acyl moiety is bound to the lipid portion in the membrane, and the enzymatic activity of Ac-SOD is the same when present in a solution or when bound to the membrane surface.

### Example 4: Binding Property of Acylated Human SOD to Cornea and Conjunctiva

To the eye of a rat, 16 µg of human-erythrocyte SOD modified with caprylic, capric, lauric or myristic acid was instilled. After 30 minutes, the eye was washed with 2 ml of physiological saline, and then the amounts of enzymes bound to the cornea and conjunctiva were measured. The results are shown in Table 1. In this Example, the four Ac-SOD's used had 5, 5, 3 and 4 acyl radicals derived from the above-mentioned fatty acids in a molecule, respectively.

**Table 1**

| Binding and Adsorption of Human Acylated SOD to Cornea and Conjunctiva of Rat | | | | | |
|---|---|---|---|---|---|
| Sites | Non-modified SOD | Acylated SOD (Ac-SOD) | | | |
| | | C₈ | C₁₀ | C₁₂ | C₁₄ |
| Cornea µg (%) | 0.096 (µg) (0.6) | 1.024 (6.4) | 0.992 (6.2) | 1.088 (6.8) | 0.912 (5.7) |
| Conjunctiva µg (%) | 0.240 (µg) (1.5) | 1.000 (6.3) | 0.848 (6.2) | 1.520 (9.5) | 1.421 (8.9) |
| (Not adsorbed) µg (%) | 0.320 (µg) (2.0) | 0.928 (5.8) | 0.904 (5.7) | 0.712 (4.5) | 1.160 (7.3) |

The eye was washed with 2 ml of physiological saline 30 minutes after the instillation of human SOD (16 µg/eye). A major portion of the SOD instilled was removed by tears prior to the washing, which was effected 30 minutes later. "Not adsorbed" means the amount of SOD in 2 ml of physiological saline.

It is obvious from Table 1 that the amounts of the acylated SOD bound to cornea and conjunctiva were as much as 4 to 10 times that of the non-modified SOD.

### Example 5: Distribution of Acylated SOD in Tissues of Rabbit Eye Having Induced Alkaline Inflammation

Non-modified SOD or lauric acid modified SOD (3 - 5 acyl radicals in a molecule), each of which had been labeled with ¹²⁵I, was instilled (200 µg; 400,000 cpm) into a rabbit eye having an induced alkaline inflammation. After 12 hours, the radioactivities at various sites were measured. Alkaline inflammation in the eye was induced by applying a circular sponge sheet (diameter: 4 mm) impregnated with 10% sodium hydroxide solution to a cornea of the rabbit for 5 seconds. The results are shown in Table 2. The activities of uncleaved enzymes are listed in parentheses.

**Table 2**

| Distribution of Acylated SOD in Tissues of Rabbit Eye Having Induced Alkaline Inflammation | | |
|---|---|---|
| Sites | Non-modified SOD | Acylated SOD (Ac-SOD) |
| Cornea | 753 (377) (cpm) | 725 (522) (cpm) |
| Conjunctiva | 51 (39) | 219 (208) |
| Lens | 0 (0) | 39 (38) |
| Iris | 36 (29) | 105 (103) |
| Sclera | 26 (19) | 89 (43) |

Non-modified SOD and Ac-SOD were instilled at 200 µg/50 µl (400,000 cpm), respectively.

Non-modified SOD was not taken up into the deep tissues of the eye, but the acylated SOD was taken up into such tissues. This denotes that the hydrophobic Ac-SOD has a good permeability for tissue cell membranes.

### Example 6: Protective Action of Acylated SOD to Keratohelcosis Induced by Endotoxin

A keratohelcosis model for a test was induced by injecting 10 µg of endotoxin within a cornea of a rabbit. To the rabbit eye, physiological saline or 200 µg of acylated SOD (same as used in Example 5) was instilled 6 times a day, and the protective actions thereof on the tissues were observed for 7 days. The permeation degrees of leukocyte to conjunctiva, cilia, aqueous humor and iris, as well as opacity of the cornea were compared. Further, total evaluations were performed. The results are listed in Table 3.

**Table 3**

| Effects of SOD on Eye Inflammation Induced by Endotoxin | | | | | | | |
|---|---|---|---|---|---|---|---|
| Sites observed | Time after endotoxin injection (day) | | | | | | |
| | 1 | 2 | 3 | 4 | 5 | 6 | 7 |
| | | | | | | | |

| (Physiological saline) | | | | | | | |
|---|---|---|---|---|---|---|---|
| Conjunctiva | 0 | 1.5 | 1 | 2 | 3 | 3 | 2 |
| Cilia | 0 | 1 | 1 | 2 | 3 | 3 | 2 |
| Cornea | 0 | 0 | 0.5 | 1 | 2 | 3 | 3 |
| Aqueous humor | 0 | 2 | 1 | 0.5 | 3 | 2 | 3 |
| Iris | 0 | 1 | 0.5 | 1 | 2 | 3 | 2 |
| Total | 0 | 5.5 | 4 | 6.5 | 16 | 14 | 12 |

| (Acylated SOD administration) | | | | | | | |
|---|---|---|---|---|---|---|---|
| Conjunctiva | 0 | 0 | 0.5 | 1 | 2 | 2 | 1 |
| Cilia | 0 | 1 | 0.5 | 0.5 | 2 | 2 | 1 |
| Cornea | 0 | 0 | 0 | 0.5 | 0.5 | 1 | 2 |
| Aqueous humor | 0 | 0.5 | 0.5 | 0 | 1 | 2 | 1.5 |
| Iris | 0 | 0 | 0.5 | 0.5 | 1 | 2 | 2 |
| Total | 0 | 1.5 | 2 | 2.5 | 6.5 | 9 | 7.5 |
| (Numerical figures 0 to 5 listed denote visual ratings from lightest to heaviest conditions.) | | | | | | | |

The administration of the acylated SOD effected a considerable cure of the diseases.

The results denote that acylated SODs embodying the invention have a good biocompatibility and permeability for tissue cell membranes, the enzymatic activity of the acylated SOD is the same whether present in a solution or bound to the membrane surface, and the acylated SOD is effective for ulcers and inflammation of the cornea.

### Preparation Example

The instillation preparation was produced by dissolving, in purified water, acylated SOD (1.5%), dipotassium glycyrrhizinate (0.1%), borax (0.3%), boric acid (0.45%), chlorobutanol (0.15%), benzalkonium chloride (0.01%) and sodium chloride (0.15%) and Filtering in a sterile condition.

Although the present invention has been described with reference to specific embodiments, various changes and modifications to these will be obvious to those skilled in the art.

## Claims

1. An acylated derivative of superoxide dismutase having the general formula 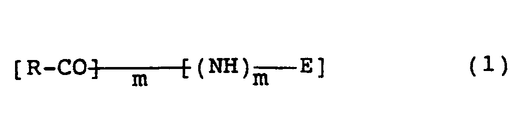 wherein R-CO- represents an acyl group of a saturated or unsaturated fatty acid having 10 to 16 carbon atoms, m is an integer of 4 to 8, and (NH)ₘ E represents a group of superoxide dismutase (NH₂)ₘ E.

2. An acylated derivative of superoxide according to claim 1, wherein the acyl group has 10 carbon atoms.

3. A composition containing an effective amount of an acylated derivative of superoxide dismutase according to claim 1 or 2, as a preventive or curing agent for a disease caused by a direct or indirect harmful action of superoxide, as a stabilizing agent for a blood product, or as a modifying agent for a surface of a medical vessel, optionally together with a suitable carrier.

4. An antiinflammatory composition, comprising an effective amount of an acylated derivative of superoxide dismutase according to claim 1 or 2, together with a pharmaceutically acceptable carrier.

5. An antiinflammatory composition according to claim 4 for treating keratohelcosis.

6. Use of an acylated derivative of superoxide dismutase, as defined in claim 1 or claim 2, in the preparation of a medicament for the treatment of keratohelcosis.

## Patentansprüche

1. Acyliertes Derivat der Superoxiddismutase der allgemeinen Formel 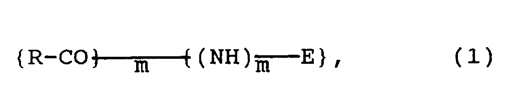 in der R-CO- eine Acylgruppe einer gesättigten oder ungesättigten Fettsäure mit 10 bis 16 Kohlenstoffatomen, m eine Zahl von 4 bis 8 und (NH)ₘ E eine Gruppe von Superoxiddismutase (NH₂)ₘ E darstellen.

2. Acyliertes Derivat der Superoxiddismutase nach Anspruch 1,
**dadurch gekennzeichnet**, daß
die Acylgruppe 10 Kohlenstoffatome enthält.

3. Zusammensetzung enthaltend eine wirksame Menge eines acylierten Derivates der Superoxiddismutase nach Anspruch 1 oder 2 als ein Präventiv- oder Heilmittel für eine, durch eine direkte oder indirekte schädigende Wirkung des Superoxids hergerufene, Erkrankung, als ein Stabilisierungsmittel für ein Blutprodukt oder als ein Modifizierungsmittel für eine Oberfläche eines medizinischen Behälters, gegebenenfalls zusammen mit einem geeigneten Träger.

4. Eine, eine wirksame Menge eines acylierten Derivates der Superoxiddismutase nach Anspruch 1 oder 2, zusammen mit einem pharmazeutisch annehmbaren Träger, enthaltende entzündungshemmende Zusammensetzung.

5. Entzündungshemmende Zusammensetzung nach Anspruch 4 zur Behandlung der Keratohelkose.

6. Verwendung eines acylierten Derivates der Superoxiddismutase nach Anspruch 1 oder 2 zur Herstellung eines Medikamentes zur Behandlung der Keratohelkose.

## Revendications

1. Un dérivé acylé de superoxyde dismutase répondant à la formule générale: 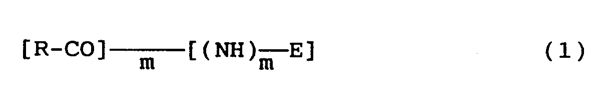 dans laquelle:
R-CO- représente un groupe acyle ou un acide gras saturé
ou insaturé renfermant de 10 à 16 atomes de carbone;
m est un nombre entier de 4 et 8; et
(NH)ₘ E représente un groupe de superoxyde dismutase (NH₂)ₘE.

2. Un dérivé acylé de superoxyde dismutase selon la revendication 1, caractérisé en ce que le groupe acyle renferme 10 atomes de carbone.

3. Une composition comprenant une quantité efficace d'un dérivé acylé de superoxyde dismutase selon la revendication 1 ou 2, comme agent de prévention ou de guérison d'une maladie occasionnée par l'action nuisible directe ou indirecte d'un superoxyde, comme agent de stabilisation d'un produit du sang, ou comme agent de modification pour de la surface d'un récipient médical, ainsi que, éventuellement, un excipient convenable.

4. Une composition anti-inflammatoire comprenant une quantité efficace d'un dérivé acylé de superoxyde dismutase selon la revendication 1 ou 2, ainsi qu'un excipient pharmaceutiquement acceptable.

5. Une composition anti-inflammatoire selon la revendication 4, pour le traitement de la kératohelcose.

6. Utilisation d'un dérivé acylé de superoxyde dismutase tel que défini dans la revendication 1 ou 2, pour la préparation d'un médicament destiné au traitement de la kératohelcose.
